# EUROPÄISCHE PATENTSCHRIFT

(11) **EP 1 395 168 B1**
(45) Veröffentlichungstag und Bekanntmachung des Hinweises auf die Patenterteilung: **12.10.2005**
(21) Anmeldenummer: 01937907.2
(22) Anmeldetag: 13.06.2001
(51) Int. Cl.: A61B 3/16

(54) **VORRICHTUNGEN ZUR BESTIMMUNG DES INNENDRUCKS EINES AUGES**
DEVICES FOR DETERMINING AN INNER PRESSURE OF THE EYE
DISPOSITIFS DE DETERMINATION DE LA PRESSION INTERNE D'UN OEIL

(43) Veröffentlichungstag der Anmeldung: 10.03.2004
(73) Patentinhaber: SIS AG Surgical Instrument Systems, 2555 Brügg b. Biel (CH)
(72) Erfinder: RATHJEN, Christian, 28197 Bremen (DE)
(74) Vertreter: Vogel, Dany
(86) Internationale Anmeldenummer: PCT/CH2001/000366
(87) Internationale Veröffentlichungsnummer: WO 2002/100260

(56) Entgegenhaltungen:
- DE-A- 3 421 701
- US-A- 4 987 898

## Beschreibung

Die vorliegende Erfindung betrifft Vorrichtungen zur Bestimmung des Innendrucks eines Auges. Irisbesondere betrifft die vorliegende Erfindung eine Vorrichtung zur Bestimmung des Innendrucks eines menschlichen Auges und eine Messsonde zur Erfassung von Messwerten zur Bestimmung des Innendrucks eines menschlichen Auges.

Der Augeninnendruck oder der sogenannte innerokulare Druck (IOD) wird durch einen geregelten Wasserfluss in der Augenkammer aufrechterhalten. Die Messung des Augeninnendrucks ist in der Ophtalmologie bei der medizinischen Diagnostik von grosser Bedeutung. Ein zu niedriger Augeninnendruck führt zur Destabilisierung des Auges. Ein zu hoher Augeninnendruck kann zu einem Glaukom (grüner Star) führen, einer breit gefächerten Gruppe von Augenerkrankungen, bei denen der IOD für eine bleibende, normale Funktion des Sehnervkopfes zu hoch ist. Der Augeninnendruck gilt als zur Zeit wichtigster und am einfachsten zu messender Parameter bei der Behandlung und Früherkennung von Glaukoma. Statistische Erfassungen liefern zum Beispiel einen mittleren Augeninnendruck, der bei den Männem 15.9 mmHg (1 mmHg = 1.33 mbar) und bei den Frauen 16.6 mmHg beträgt. Der Augeninnendruck unterliegt sowohl täglichen Schwankungen von 3 bis 6 mmHg als auch Schwankungen durch den Blutdruck (Puls) von etwa 1.5 mmHg. Obwohl der Augeninnendruck von Individuum zu Individuum verschieden ist, werden oft 21 mmHg als Grenzwert gesetzt, bei dem zumindest eine weitere medizinische Abklärung einer Glaukomagefährdung als notwendig erachtet wird. Zudem wird auch eine tägliche Schwankung des IOD von 10 mmHg als pathologisch beurteilt. In neusten Ansätzen wird selbst die Veränderung der durch den Puls verursachten kurzzeitigen Schwankungen des IOD bei Glaukoma untersucht.

Der Augeninnendruck wird durch sogenannte Tonometer gemessen. In den bekannten Tonometrie-Verfahren wird der Druck durch Kraftmessung bei bekannter Fläche oder durch Flächenmessung bei bekannter Kraft bestimmt. Es gibt auch Tonometrie-Verfahren, bei denen ein externer Druck auf das Auge aufgebracht und der aufgebrachte Druck gemessen wird. Des weiteren gibt es nicht kontaktierende Tonometer, die mittels eines Luftstosses eine Abflachung der Hornhaut erzeugen und dadurch den IOD messen können. Das zur Zeit als Genaustes betrachtete und weit verbreitete Goldmann Tonometer basiert auf einer Kraftmessung bei konstanter Kontaktfläche (bei planer Kontaktierung Applanationsfläche genannt). Beim Goldmann Tonometer wird die Grösse der Applanationsfläche so gewählt, dass sich die durch die Hornhautsteifigkeit verursachten Kräfte und die Adhäsionskräfte, die durch den Tränenminiskus zwischen der Applanationsfläche und dem Auge verursacht werden, aufheben, was für kreisförmige Applanationsflächen bei einem Durchmesser von 3 bis 4 mm gilt. Beim Goldmann Tonometer wird die vordefinierte Grösse der Applanationsfläche eingestellt, indem die auf die Applanationsfläche ausgeübte Kraft so lange erhöht wird, bis sich beim Hindurchschauen durch den mit einem Doppelprisma versehenen gläsernen Applanationskörper zwei Halbkreise berühren. Der Augeninnendruck IOD ergibt sich dann aus dem Quotienten F/A der gemessenen, auf die Applanationsfläche ausgeübten Kraft F und der eingestellten, vordefinierten Applanationsfläche A. Die Goldmann Tonometer werden mit einer Spaltlampe verwendet und sind typischerweise als Standgeräte ausgeführt, so dass der Patient sitzen muss und die Selbsttonometrie durch den Patienten nicht möglich ist. Handausführungen des Goldmann Tonometers sind gerätetechnisch noch aufwendiger realisiert. Der gerätetechnische Aufwand ist beim Goldmann Tonometer vor allem dann sehr gross, wenn der Messprozess, wie für die Selbsttonometrie durch den Patienten nötig, automatisiert wird, da die Applanationsfläche automatisch bestimmt und eingestellt werden muss.

Eine neuere Vorrichtung zur Bestimmung des Augeninnendrucks, die als einfach aufzusetzendes Kontaktglas ausgebildet ist und sich deshalb scheinbar für die Selbsttonometrie eignen könnte, wird in der veröffentlichten Patentanmeldung WO 00/71982 beschrieben. Das Kontaktglas gemäss WO 00/71982 weist eine konkave Ausnehmung auf, deren Flächenkontur der menschlichen Augenwölbung angepasst ist. Im Scheitelpunkt der Ausnehmung ist eine durch eine Membran abgedichtete Bohrung angebracht. Die Bohrung ist Teil eines flüssigkeitsgefüllten Kammersystems, das einen auf die Membran ausgeübten Druck auf eine im Kammersystem befindliche Druckmesseinheit überträgt, wobei die Flüssigkeit als Druckübertragungsmittel dient. Bedingt durch die Form der Vorrichtung gemäss WO 00/71982 kann der Augeninnendruck beim nicht vorschriftsmässigen Aufsetzen auf das Auge (leicht und ohne Kraftaufwendung) und periodischem Abheben der Vorrichtung vom Auge allerdings stark beeinflusst werden, so dass sich Messwerte ergeben, die zur Bestimmung eines inkorrekten IOD führen. Dies erschwert massgeblich die Selbsttonometrie durch den Patienten.

In der Patentanmeldung DE 3421701 wird eine Vorrichtung zur Bestimmung des Augeninnendrucks beschrieben, welche mehrere Drucksensoren umfasst, deren Messwerte zur Berechnung des Augeninnendrucks verwendet werden. Um den Einfluss des gegenüber dem Gegendruck auf der Applanationsfläche wesentlich geringeren Gegendrucks seitlich von der Applanationsfläche, der durch die sich dort ansammelnde Tränenflüssigkeit verursacht wird, weitgehendst auszuschalten, umfasst die Vorrichtung gemäss DE 3421701 zudem einen Schwellenwertgeber.

Um ein korrektes Messresultat zu erhalten, müssen auch die bekannten Tonometer sehr präzise, insbesondere senkrecht zur Hornhaut, und sorgfältig appliziert werden. Da einige der bekannten Tonometer nur den momentanen Wert des IOD angeben, müssen zudem mehrere Messungen durchgeführt werden, um den mittleren IOD zu bestimmen. Die bekannten Tonometer liefern zudem im Vergleich untereinander unterschiedliche Messwerte. Die Dicke, die Steifigkeit und die Form der Hornhaut beeinflussen bei vielen Verfahren die Messwerte des IOD. Zur Berücksichtigung der Hornhautdicke wird unter anderem versucht, die IOD-Messung mit einer Hornhautdickenmessung zu kombinieren.

Es ist eine Aufgabe dieser Erfindung, eine Vorrichtung zur Bestimmung des Innendrucks eines Auges vorzuschlagen, welche mindestens gewisse der oben beschriebenen Nachteile des Stands der Technik nicht aufweisen und welche insbesondere eine ortsaufgelöste Bestimmung des Augeninnendrucks ermöglichen.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Insbesondere werden diese Ziele durch die Erfindung dadurch erreicht, dass die Vorrichtung zur Bestimmung des Augeninnendrucks mehrere Drucksensorelemente umfasst, die mittelbar oder unmittelbar auf das Auge aufgesetzt werden, und dass sie Verarbeitungsmittel zur Bestimmung des Augeninnendrucks aus den durch die einzelnen Drucksensorelemente gemessenen Sensordruckwerten umfasst. Die Drucksensorelemente sind vorzugsweise in einem Drucksensor-Array angeordnet, der mittelbar oder unmittelbar auf das Auge aufgesetzt wird, wobei der Drucksensor-Array mindestens eine Zeile vorzugsweise aber mehrere Zeilen von den Drucksensorelementen umfasst. Der Vorteil, mehrere Drucksensorelemente, insbesondere einen Drucksensor-Array, zur Bestimmung des Augeninnendrucks zu verwenden, besteht insbesondere darin, dass der Augeninnendruck ortsaufgelöst bestimmt werden kann. Dadurch können Ungleichmässigkeiten in der örtlichen Druckverteilung erkannt werden und beispielsweise eine schlechte Applikation, das heisst ein unkorrektes Aufsetzen des Drucksensor-Arrays auf das Auge, als ungültige Messung erkannt werden. Insbesondere kann eine zu starke oder zu schwache Kontaktierung (bei planer Kontaktierung Applanation genannt) des Auges beim Aufsetzen des Drucksensor-Arrays erkannt werden und dem Benutzer angezeigt werden. Da weder eine vordefinierte Grösse der Kontaktfläche (bei planer Kontaktierung Applanationsfläche) noch eine vordefinierte Andruckkraft eingestellt werden muss, müssen Patienten auch weniger ruhig gestellt und ausgerichtet werden. Zudem wird insbesondere im Fall einer ebenen Anordnung der Drucksensorelemente eine besonders einfache Applikation der Vorrichtung ermöglicht, da bloss eine ebene Fläche (Drucksensor-Array) in Kontakt mit einem Kugelabschnitt (Hornhaut) gebracht werden muss und nicht wie in einigen Verfahren des Stands der Technik auf eine Zentrierung der Kontaktfläche und auf eine senkrechte Applikation der Vorrichtung geachtet werden muss. Ein weiterer Vorteil besteht darin, dass die Verwendung eines Drucksensor-Arrays die Bestimmung des Augeninnendrucks ohne bewegte mechanische Teile ermöglicht.

Die Verarbeitungsmittel sind vorzugsweise so ausgebildet, dass sie den Augeninnendruck aus der Summe der gemessenen und beispielsweise gewichteten Sensordruckwerte und der Anzahl zur Summe beitragender Drucksensorelemente bestimmen. Da der Augeninnendruck durch eine Division der Summe der gemessenen Sensordruckwerte durch die Anzahl beitragender (aktiver) Drucksensorelemente berechnet wird, muss der Drucksensor-Array nicht mit sämtlichen seiner Drucksensorelemente auf dem Auge aufliegen. Daraus ergibt sich der Vorteil, dass sich verschiedene Kontaktzustände zur Bestimmung des Augeninnendrucks eignen und beispielsweise ovale Kontaktflächen, wie sie bei Astigmatismus auftreten, die Bestimmung des Augeninnendrucks nicht verfälschen.

Erfindungsgemäss umfasst die Vorrichtung Filtermittel zum Ausschliessen gewisser gemessener Sensordruckwerte von der Summierung gemäss vordefinierten Kriterien. Die Vorrichtung umfasst Filtermittel zum Ausschliessen von denjenigen gemessenen Sensordruckwerten von der Summierung, die mindestens mit einem definierten Differenzwert über einem Sensordruckwert liegen, der von denjenigen Drucksensorelementen gemessen wird, die im aufgesetzten Zustand der Vorrichtung in einem inneren Bereich einer Kontaktfläche liegen, auf welcher die Vorrichtung mittelbar oder unmittelbar auf dem Auge aufliegt. Dadurch können einzelne Sensordruckwerte, die die Bestimmung des Augeninnendrucks ungünstig beeinflussen, von der Berechnung des Augeninnendrucks ausgeschlossen werden. Zum Beispiel können Sensordruckwerte, die von Drucksensorelementen am äusseren Rand der Kontaktfläche gemessen werden und die durch die Hornhautsteifigkeit und/oder durch Adhäsionskräfte des Tränenminiskus zwischen dem Auge und dem betreffenden Drucksensorelement beeinflusst werden, von der Bestimmung des Augeninnendrucks ausgeschlossen werden. Es können auch Sensordruckwerte ausgeschlossen werden, die ansonsten die Bestimmung des Augeninnendrucks wegen variabler Hornhautdicke des betreffenden Auges verfälschen würden, beispielsweise bedingt durch eine lokale Homhautverdickung oder -vertiefung. Es ist auch möglich, Ungleichmässigkeiten, die durch die Hornhautsteifigkeit verursacht werden, zu detektieren und zur Kompensation der Einflüsse der Hornhautsteifigkeit auszunützen. Das Ausschliessen gewisser gemessener Sensordruckwerte kann auch dann vorteilhaft sein, wenn der verwendete Drucksensor-Array eine grobe Ortsauflösung aufweist, das heisst, wenn der Drucksensor-Array aus wenigen Drucksensorelementen besteht, die den Druck jeweils auf einer relativ grossen Kontaktfläche messen, so dass einzelne Drucksensorelemente am äusseren Rand der Kontaktfläche nur teilweise auf dem Auge aufliegen.

In einer Ausführungsvariante umfasst die Vorrichtung Speichermittel zur Speicherung der gemessenen Sensordruckwerte und zur Speicherung mehrerer bestimmter Werte des Augeninnendrucks. Dadurch kann die Erfassung (Messung) der Sensordruckwerte und die Auswertung der erfassten Sensordruckwerte in verschiedenen, zeitlich verschobenen Schritten durchgeführt werden, was beispielsweise auch eine Selbsttonometrie durch den Patienten erleichtern kann. Die Speicherung der gemessenen Sensordruckwerte erleichtert auch die Berechnung zeitlicher Mittelwerte der gemessenen Sensordruckwerte. Zudem kann die Amplitude der durch den Puls des Blutdrucks verursachten Druckschwankung des IOD erfasst werden. Die Speicherung von mehreren bestimmten Augeninnendruckwerten, vorzugsweise jeweils zusammen mit zugeordneten Zeitangaben, ermöglicht zudem den Vergleich von Augeninnendruckwerten, die zu verschiedenen Zeitpunkten bestimmt wurden.

In einer Ausführungsvariante umfasst die Vorrichtung Darstellungsmittel zur grafischen und insbesondere ortsaufgelösten Darstellung der gemessenen und/oder gespeicherten Sensordruckwerte. Dadurch können Druckverteilungsprofile entlang einer Linie durch die Kontaktfläche und/oder zweidimensionale Druckverteilungsprofile über die gesamte Kontaktfläche, beispielsweise Druckverteilungsmatrizen, dargestellt und analysiert werden. Durch die grafische Darstellung gespeicherter Sensordruckwerte können auch Sequenzen von Druckverteilungsprofilen und Druckverteilungsmatrizen dargestellt und analysiert werden.

In einer Ausführungsvariante umfasst die Vorrichtung Kommunikationsmittel zur Fernübermittlung der bestimmten Augeninnendruckwerte. Dadurch wird beispielsweise ermöglicht, dass die von einem Patienten durch Selbsttonometrie bestimmten Augeninnendruckwerte zur Erfassung und Auswertung an einen zuständigen Arzt übermittelt werden können.

Vorzugsweise ist der Drucksensor-Array als mikroelektromechanisches System (MEMS) ausgeführt. Dadurch wird ein hoher Integrationsgrad und dadurch ein hoher Miniaturisierungsgrad ermöglicht, so dass kompakte, tragbare Geräte mit einer hohen Ortsauflösung realisiert werden können.

In einer Ausführungsvariante ist der Drucksensor-Array in eine Messsonde eingebaut, die über eine kontaktbehaftete oder eine kontaktlose Schnittstelle mit einer Auswertungseinheit verbunden ist. Dadurch können besonders kompakte Messsonden realisiert werden, die über entsprechende Schnittstellen und programmierte Softwaremodule mit herkömmlichen Verarbeitungs- und Anzeigeeinheiten kombiniert werden können, beispielsweise mit Palmtop-, Laptop- oder Personal-Computern oder mit Mobilfunktelefonen.

Nachfolgend wird eine Ausführung der vorliegenden Erfindung anhand eines Beispieles beschrieben. Das Beispiel der Ausführung wird durch folgende beigelegte Figuren illustriert:
Figur 1 zeigt ein Blockdiagramm, das schematisch eine Vorrichtung zur Bestimmung des Augeninnendrucks mit einem Drucksensor-Array, Verarbeitungsmitteln und einer Anzeige illustriert.
Figur 2 zeigt eine schematische Darstellung der Vorrichtung zur Bestimmung des Augeninnendrucks, in der die Messsonde mit dem Drucksensor-Array mit einer Anzeige versehen ist.
Figur 3 zeigt eine schematische Darstellung einer weiteren Ausführungsvariante der Vorrichtung zur Bestimmung des Augeninnendrucks, in der die Messsonde über eine kontaktbehaftete Schnittstelle mit einer Auswertungseinheit verbunden ist.
Figur 4 zeigt eine schematische Darstellung einer Vorrichtungsanordnung zur Bestimmung des Augeninnendrucks, in der die Messsonde über eine kontaktlose Schnittstelle mit der Auswertungseinheit verbunden ist.
Figur 5 zeigt ein schematisches Beispiel eines Druckverteilungsprofils entlang einer Linie durch die Fläche, auf der die Messsonde auf dem Auge aufliegt (das heisst durch die Kontaktfläche, oder bei planer Kontaktierung durch die Applanationsfläche).

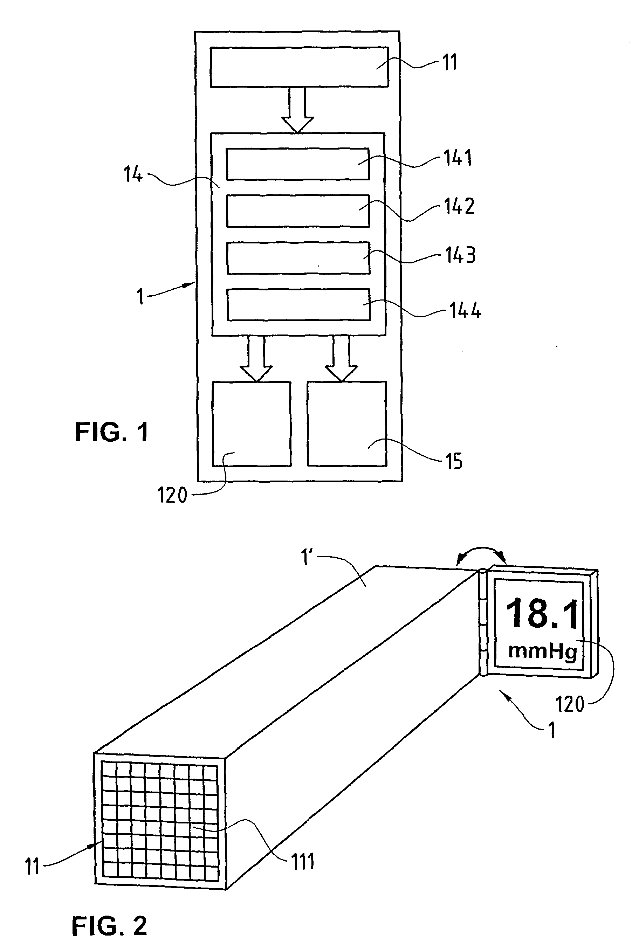

In der Figur 1 bezieht sich das Bezugszeichen 1 auf die Vorrichtung zur Bestimmung des Augeninnendrucks, die im nachfolgenden Text auch als Messvorrichtung 1 bezeichnet wird. Die Messvorrichtung 1 umfasst mehrere Drucksensorelemente 111, die beispielsweise entlang einer Linie, einer Geraden oder kreisförmig, aber vorzugsweise matrixförmig, in einem Drucksensor-Array 11 angeordnet sind. Die Drucksensorelemente 111 können auch als Drucksensorringe ausgeführt und konzentrisch angeordnet sein. Drücke, die durch die Drucksensorelemente 111 aufgenommen werden, führen zu Verformungen sensitiver Elemente der Drucksensorelemente 111, die je nach Ausführungsform der Drucksensorelemente 111, respektive ihrer sensitiven Elemente, beispielsweise kapazitiv, piezoelektrisch, resistiv, optisch, durch Veränderungen von Resonanzfrequenzen oder durch andere geeignete Verfahren detektiert werden. Die sensitiven Elemente der Drucksensorelemente 111, mittels derer der Druck detektiert wird, werden beispielsweise unmittelbar über Sensorflächen auf die Hornhaut 21 oder die Sklera des Auges 2 aufgesetzt, es ist aber auch möglich, dass die Sensorflächen über geeignete Verbindungen, zum Beispiel fluidische Verbindungen, mit den sensitiven Elementen der Drucksensorelemente 111 verbunden sind. Fluidische Verbindungen können statischer oder dynamischer Natur sein. Bei dynamischen Verbindungen wird beispielsweise ein Luft- oder Flüssigkeitsstrom zu einer Öffnung befördert, die im mittel- oder unmittelbaren Kontakt zur Hornhaut steht. Bei der Annäherung und dem Kontakt mit der Hornhaut wird der Strömungswiderstand an der Öffnung erhöht. Der sich aufbauende Gegendruck an der Hornhaut kann zur Messung des IOD genutzt werden.

An dieser Stelle soll festgehalten werden, dass das Aufsetzen der Drucksensorelemente 111, respektive des Drucksensor-Arrays 11, auf das Auge 2 mittelbar oder unmittelbar erfolgen kann. Neben der Bestimmung des Augeninnendrucks durch unmittelbares Aufsetzen des Drucksensor-Arrays 11 auf das Auge 2 kann die Bestimmung des Augeninnendrucks auch durch Aufsetzen des Drucksensor-Arrays 11, respektive der Drucksensorelemente 111, auf eine auf der Hornhaut 21 angebrachte Kontaktlinse oder durch Aufsetzen auf das geschlossene Augenlid durchgeführt werden. Die Bestimmung des Augeninnendrucks durch eine Kontaktlinse oder ein Augenlid hindurch hat den Vorteil, dass das Auge 2 nicht durch den Kontakt mit den Drucksensorelementen 111 beeinträchtigt wird.

Der Drucksensor-Array 11 ist vorzugsweise als mikroelektromechanisches System ausgeführt. Es sind mikroelektromechanische Drucksensor-Arrays 11 verfügbar, die gemeinsam mit Verarbeitungseinheiten, beispielsweise mit Analog/Digital-Wandlern zur Digitalisierung von Messwerten, auf einem Chip integriert sind. Solche Drucksensor-Array-Chips werden beispielsweise mittels CMOS-kompatiblen Verfahrenstechniken hergestellt. Drucksensor-Arrays werden insbesondere auf dem Gebiet des "Tactile Sensing" entwickelt und eingesetzt, zum Beispiel in der Robotik. Weitere Anwendungen von Drucksensor-Arrays finden sich in der Biometrie, z.B. bei der Erkennung von Fingerabdrücken oder bei der Messung von Druckprofilen auf der Fusslauffläche. Von Toyota wurde beispielsweise ein Drucksensor-Array-Chip mit 32X32 Drucksensorelementen 111, einer lateralen Auflösung von 250 µm und einer Gesamtgrösse von 10x10 mm in CMOS-Technologie hergestellt. Wie später anhand der Figuren 2 bis 5 beschrieben wird, ist der Drucksensor-Array 11 zum Aufsetzen auf das Auge 2 in eine Messsonde 1' eingebaut. Um sterile Messbedingungen zu erhalten und um das Auge und den Drucksensor-Array 11 zu schützen, wird der Drucksensor-Array 11 beispielsweise mittels einer wegwerfbaren Membran abgedeckt. Der Fachmann wird verstehen, dass der Drucksensor-Array 11 auch bloss mit einer einzigen Zeile von Drucksensorelementen 111 ausgeführt werden kann. Es ist auch denkbar, die Drucksensorelemente 111 des Drucksensor-Arrays 11 nicht flach sondern auf einem gewölbten Träger anzuordnen, dessen Kontur beispielsweise der menschlichen Augenwölbung angepasst ist. Die Drucksensorelemente 111 sind vorzugsweise regelmässig im Drucksensor-Array 11 angeordnet; die Abstände zwischen den einzelnen Drucksensorelementen 111 können allerdings auch unterschiedlich sein.

Wie in der Figur 1 schematisch dargestellt wird, werden die von den Drucksensorelementen 111 des Drucksensor-Arrays 11 gemessenen Drucksensorwerte zur weiteren Auswertung an die Verarbeitungsmittel 14 weitergeleitet. Falls Drucksensor-Arrays 11 eingesetzt werden, die Drucksensorelemente 111 umfassen, die zusätzlich zu Normalkräften auch Scherkräfte aufnehmen, können zusätzlich zu den gemessenen Drucksensorwerten auch gemessene Scherkräfte (Schubkräfte) zur Auswertung an die Verarbeitungsmittel 14 geleitet werden.

Die Verarbeitungsmittel 14 umfassen Speichermittel 141, insbesondere elektronische Datenspeicher, in denen die gemessenen Drucksensorwerte gespeichert werden. Die Speichermittel 141 umfassen beispielsweise elektronische Datenspeicher, die gemeinsam mit dem Drucksensor-Array 11 auf einem Chip integriert sind.

Die Verarbeitungsmittel 14 umfassen Filtermittel 142, die gewisse der gemessenen Drucksensorwerte gemäss vordefinierten Kriterien von der weiteren Verarbeitung ausschliessen. Es werden insbesondere Drucksensorwerte von der weiteren Verarbeitung ausgeschlossen, die die Bestimmung des Augeninnendrucks verfälschen würden. Die Filtermittel 142 sind entweder hardwaremässig ausgeführt, beispielsweise als integrierte Schaltkreise, oder sie sind als programmierte Softwaremodule zur Steuerung eines Prozessors ausgeführt. Die Funktionsweise der Filtermittel 142 entspricht bekannten Algorithmen aus der digitalen Signal- und Bildverarbeitung, wobei in Analogie zur Bildverarbeitung die Matrix oder Zeile der gemessenen Sensordruckwerte einer Bildmatrix oder Bildzeile von Pixels (Picture Element) mit digitalisierten Grauwerten entspricht. Sowohl für beliebige Zwecke programmierbare DSP- (Digital Signal Processor) oder Bildverarbeitungsprozessor-Chips als auch in Chips integrierte Verarbeitungsalgorithmen sind verfügbar.

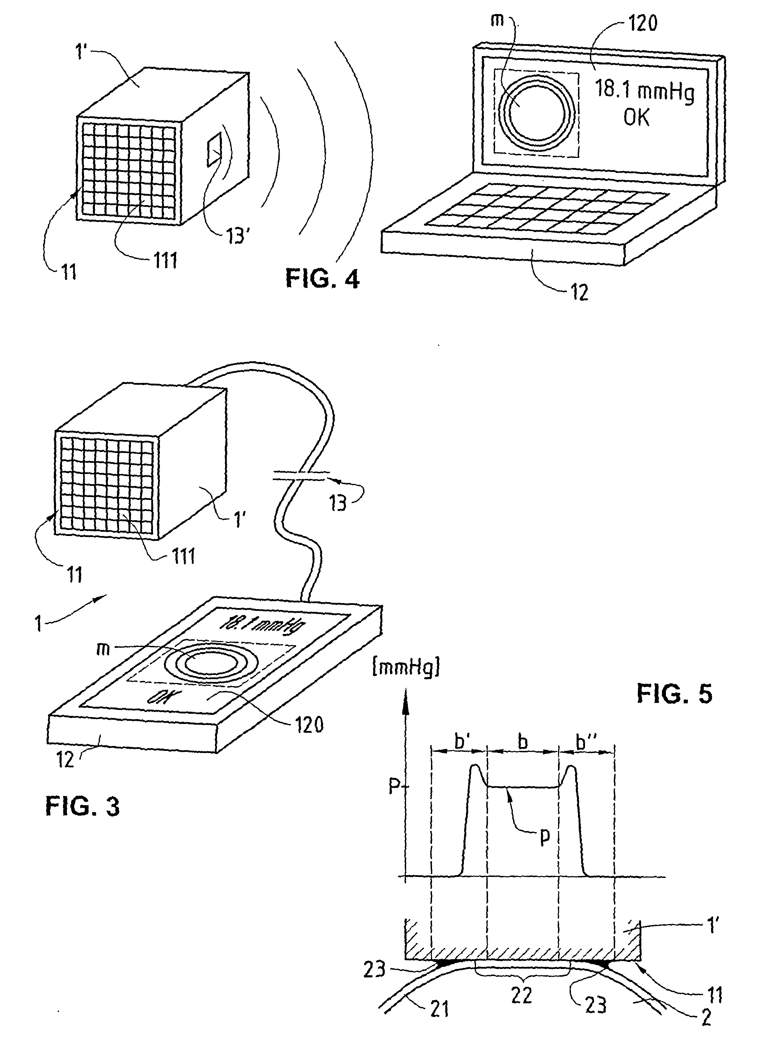

Eine der Funktionen der Filtermittel 142 soll anhand der Figur 5 erläutert werden. Im unteren Teil der Figur 5 ist schematisch eine Messsonde 1' dargestellt, die mit ihrem Sensor-Array 11 auf der Hornhaut 21 eines Auges 2 aufliegt. Die Fläche, auf dem der Sensor-Array 11 auf der Hornhaut 21 aufliegt, die Kontaktfläche (oder Applanationsfläche bei planer Auflage), wird in der Figur 5 mit dem Bezugszeichen 22 bezeichnet. Im oberen Teil der Figur 5 ist beispielhaft ein Druckverteilungsprofil p dargestellt, das dem Verlauf der erfassten Druckwerte entlang einer Linie durch die Kontaktfläche 22 oder entlang einer Zeile des auf dem Auge 2 aufliegenden Drucksensor-Arrays 11 entspricht. Die mit b' und b" bezeichneten Bereiche des Druckverteilungsprofils p umfassen die Sensordruckwerte, die von denjenigen Drucksensorelementen 111 des Drucksensor-Arrays 11 gemessen werden, die unter dem Einfluss der Hornhautsteifigkeit und/oder unter dem Einfluss der Adhäsionskraft stehen, die durch den zwischen der Hornhaut 21 und dem Drucksensor-Array 11 liegenden Tränenminiskus 23 verursacht wird. Die durch den Tränenminiskus 23 beeinflussten Drucksensorelemente 111 liegen im Bereich ausserhalb der Kontaktfläche 22. Der mit b bezeichnete Bereich des Druckverteilungsprofils p umfasst die Sensordruckwerte, die von denjenigen Drucksensorelementen 111 des Drucksensor-Arrays 11 gemessen werden, die im inneren Bereich der Kontaktfläche 22 liegen und die nicht oder nur geringfügig durch die Hornhautsteifigkeit und/oder den Tränenminiskus 23 beeinflusst werden. Da die Bereiche b' und b" des Druckverteilungsprofils p die auf den gemessenen Sensordruckwerten basierende Berechnung des Augeninnendrucks verfälschen können, können die in diesen Bereichen liegenden Sensordruckwerte durch die Filtermittel 142 von der weiteren Verarbeitung ausgeschlossen werden. Die entsprechende Filterfunktion kann beispielsweise durch Filterung der Sensordruckwerte, die mindestens mit einem definierten Differenzwert über, respektive unter dem im Bereich b vorherrschenden Sensordruckwert P liegen, realisiert werden. Die entsprechende Filterfunktion kann auch durch Filterung der Sensordruckwerte, die innerhalb eines vordefinierten oder dynamisch bestimmten geometrischen Bereichs b erfasst werden, realisiert werden. Entsprechende Filterfunktionen können auch zum Ausschliessen von Sensordruckwerten eingesetzt werden, die durch Drucksensorelemente 111 gemessen werden, die sich im äusseren Bereich der Kontaktfläche 22 befinden und die nur teilweise auf der Hornhaut 21 aufliegen. Ein teilweises Aufliegen von Drucksensorelementen 111 ist beispielsweise bei Drucksensor-Arrays 11 mit grober Ortsauflösung möglich, dessen Drucksensorelemente 111 beispielsweise eine Elementbreite von einem oder mehr als einem Millimeter aufweisen.

Die Filtermittel 142 können auch Funktionen aufweisen, um gemessene Sensordruckwerte auszuschliessen, die ansonsten die Berechnung des Augeninnendrucks wegen variabler Hornhautdicke des betreffenden Auges 2 verfälschen würden, beispielsweise bedingt durch eine lokale Hornhautverdickung oder -vertiefung, zum Beispiel verursacht durch chirurgische Eingriffe. Es kann zudem vorteilhaft sein, dass wenigstens gewisse Filterfunktionen der Filtermittel 142 durch den Benutzer wahlweise ein- und ausschaltbar sind. Die Filtermittel 142 können auch so ausgeführt sein, dass sie dem Benutzer ermöglichen, Interessensbereiche (areas of interest) festzulegen und zu wählen.

Die Verarbeitungsmittel 14 umfassen Druckberechnungsmittel 143, die aus den von den Filtermitteln 142 nicht ausgeschlossenen gemessenen Sensordruckwerten den Augeninnendruck berechnen. Dabei wird die Summe dieser Sensordruckwerte, die beispielsweise gewichtet werden, durch die Anzahl der Drucksensorelemente 111 dividiert, die zur Summe beitragen, das heisst die Anzahl aktiver Drucksensorelemente 111, die einen gemessenen Sensordruckwert angeben und deren Sensordruckwert nicht durch die Filtermittel 142 von der weiteren Verarbeitung ausgeschlossen wurde. Die Druckberechnungsmittel 143 sind entweder hardwaremässig ausgeführt, beispielsweise als integrierte Schaltkreise, oder sie sind als programmierte Softwaremodule zur Steuerung eines Prozessors ausgeführt. Zur Kalibrierung der gemessenen Sensordruckwerte in normierte Druckwerte umfassen die Verarbeitungsmittel 14 zudem Kalibrierfunktionen und je nach Ausführung dazugehörende Kalibriertabellen. Die Verarbeitungsmittel 14 umfassen beispielsweise auch eine Kalibrierfunktion, die es dem Benutzer ermöglicht, die Drucksensorelemente 111 wahlweise zu kalibrieren, beispielsweise kann eine Kalibrierung gewählt werden, die Messwerte liefert, die denjenigen eines Goldmann Tonometers entsprechen.

Die Verarbeitungsmittel 14 umfassen Darstellungsmittel 144 zur grafischen Darstellung der gemessenen Sensordruckwerte auf einer Anzeige 120, beispielsweise eine LCD-Anzeige. Die Darstellungsmittel 144 sind vorzugsweise als programmierte Softwaremodule zur Steuerung eines Prozessors ausgeführt. Die Darstellungsmittel 144 umfassen beispielsweise Funktionen zur Erzeugung und Darstellung von Druckverteilungsprofilen p gemäss der Figur 5 oder von zweidimensionalen Druckverteilungsprofilen m (bei mehrzeiligen Drucksensor-Arrays 11), die das zweidimensionale Druckprofil des Drucksensor-Arrays 11 wiedergeben. Die zweidimensionalen Druckverteilungsprofile m sind beispielsweise Druckverteilungsmatrizen in denen die Matrix der gemessenen Sensordruckwerte, beispielsweise mittels den Sensordruckwerten entsprechenden, unterschiedlichen Grau- oder Farbwerten, ortsaufgelöst bildlich dargestellt wird (siehe Figuren 3 und 4). Die Auflösung und Darstellung der zweidimensionalen Druckverteilungsprofile m brauchen jedoch nicht mit der Anzahl Drucksensorelemente 111 und/oder der Struktur des Drucksensor-Arrays 11 übereinzustimmen. Die Darstellungsmittel 144 umfassen beispielsweise auch Funktionen zur grafischen Darstellung des zeitlichen Verlaufs des berechneten Augeninnendrucks oder zur grafischen Darstellung von Sequenzen von erfassten und/oder gespeicherten Zeilen oder Matrizen von gemessenen Sensordruckwerten. Die Darstellungsmittel 144 können beispielsweise auch so mit den Filtermitteln 142 kombiniert werden, dass wahlweise Sensordruckwerte, die durch die Filtermittel 142 von der Berechnung des Augeninnendrucks ausgeschlossen werden, bei der Darstellung von Druckverteilungsprofilen p, m (Druckverteilungsmatrizen) miteinbezogen und beispielsweise optisch hervorgehoben werden. Dadurch können beispielsweise Augenunregelmässigkeiten, zum Beispiel variable Hornhautdicken, auf der Anzeige 120 sichtbar gemacht werden.

Zu Steuerungs- und Bedienungszwecken umfasst die Messvorrichtung 1 zudem Bedienungselemente, beispielsweise Tasten, mit denen beispielsweise auch auf der Anzeige 120 angezeigte Menus bedient werden können.

Die Messvorrichtung 1 kann beispielsweise in verschiedenen durch den Benutzer wählbaren Betriebsmodi betrieben werden. In einem ersten Betriebsmodus werden die durch die Drucksensorelemente 111 des Drucksensor-Arrays 11 gemessenen Sensordruckwerte erfasst, in den Speichermitteln 141 gespeichert und daraus unmittelbar der Augeninnendruck berechnet und beispielsweise zusammen mit dem entsprechenden Druckverteilungsprofil p, m (z.B. eine Druckverteilungsmatrix) auf der Anzeige 120 dargestellt. In einem weiteren Betriebsmodus werden automatisch mehrere sequentielle Bestimmungen des Augeninnendrucks gemäss dem ersten Betriebsmodus vorgenommen und davon der Mittelwert als resultierender Augeninnendruck angezeigt. In einem weiteren Betriebsmodus werden die durch die Drucksensorelemente 111 des Drucksensor-Arrays 11 gemessenen Sensordruckwerte kontinuierlich erfasst, in den Speichermitteln 141 gespeichert und daraus unmittelbar der aktuelle und/oder durchschnittliche Augeninnendruck berechnet und beispielsweise zusammen mit dem zeitlichen Verlauf des Augeninnendrucks auf der Anzeige 120 dargestellt. Dadurch können beispielsweise auch Druckschwankungen, die durch den Puls des Blutdrucks verursacht werden, sichtbar gemacht werden. Insbesondere zur Unterstützung des Benutzers bei der Applikation der Messvorrichtung 1 kann das Druckverteilungsprofil p, m (z.B. eine Druckverteilungsmatrix) der laufend gemessenen aktuellen Sensordruckwerte auf der Anzeige 120 dargestellt werden, beispielsweise ohne Zwischenspeicherung in den Speichermitteln 141, so dass der Benutzer ein Echtzeit-Feedback des durch das Aufsetzen des Drucksensor-Arrays 11 auf dem Auge 2 erzeugten Kontakts (Applanation) erhält. Dem Benutzer kann beispielsweise eine korrekte Applikation angezeigt werden (OK) und ihm die Möglichkeit gegeben werden, die Messung gemäss einem gewählten Betriebsmodus beispielsweise auf Knopfdruck aktiv auszulösen.

Die berechneten Werte des Augeninnendrucks können in den Speichermitteln 141 auch längerfristig gespeichert werden, beispielsweise automatisch oder wahlweise, auf Befehl des Benutzers. Berechnete und gespeicherte Werte können untereinander verglichen und beispielsweise an einen Arzt übermittelt werden. Zur späteren Auswertung werden die berechneten Werte des Augeninnendrucks vorzugsweise zusammen mit Zeitangaben über den Zeitpunkt der Augeninnendruckbestimmung, die beispielsweise die Uhrzeit und das Datum umfassen und die beispielsweise durch ein nicht dargestelltes Zeiterfassungsmodul der Vorrichtung 1 geliefert werden, in den Speichermitteln 141 gespeichert. In den Speichermitteln 141 können zudem weitere Informationen den Augendruckwerten zugeordnet abgespeichert werden, beispielsweise Angaben über die Messbedingungen, den Zustand des Patienten oder Medikamente, die vom Patienten eingenommen wurden. Diese Zusatzinformationen können beispielsweise mittels gesprochener Sprache oder mittels den oben erwähnten Bedienungselementen eingegeben werden. Zur Übermittlung der berechneten Werte des Augeninnendrucks umfasst die Vorrichtung 1 beispielsweise ein Kommunikationsmodul 15, das zum Beispiel für die Datenübertragung über Mobilfunknetze, beispielsweise GSM- (Global System for Mobile Communication) oder UMTS-Netze (Universal Mobile Telephone System), oder Festnetze, beispielsweise PSTN- (Public Switched Telephone Network), ISDN-(Integrated Services Digital Network) oder IP-Netze (Internet Protocol), ausgeführt ist. Gespeicherte Werte können auf Befehl des Benutzers und/oder automatisch, beispielsweise nach einer vordefinierten Zeit, gelöscht werden.

In der Figur 2 ist schematisch eine kompakte Ausführungsvariante der Messvorrichtung 1 dargestellt, die besonders gut für die Selbsttonometrie durch den Patienten geeignet ist. In dieser Ausführungsvariante ist die gesamte Messvorrichtung 1 mit dem Drucksensor-Array 11 und den Verarbeitungsmitteln 14, beispielsweise batteriegespeist, in einer mobilen, tragbaren Messsonde 1' untergebracht, wobei die Anzeige 120 so angeordnet ist, dass sie durch den untersuchenden Arzt oder durch den Patienten selber abgelesen werden kann, beispielsweise indem sie, wie in der Figur 2 illustriert, schwenkbar ausgestaltet ist.

In der Figur 3 ist schematisch eine Ausführungsvariante der Messvorrichtung 1 dargestellt, in der die funktionellen Module der Messvorrichtung 1 auf die Messsonde 1' und die Auswertungseinheit 12 aufgeteilt sind. Wie in der Figur 3 dargestellt ist, ist die Messsonde 1', die den Drucksensor-Array 11 umfasst, über eine kontaktbehaftete Schnittstelle 13, beispielsweise eine Kabelverbindung mit einer Speiseleitung, mit der Auswertungseinheit 12, die die Anzeige 120 umfasst, verbunden. Die Verarbeitungsmittel 14 können auf verschiedene Weise auf die Messsonde 1' und die Auswertungseinheit 12 aufgeteilt werden. Zusätzlich zum Drucksensor-Array 11 umfasst die Messsonde 1' beispielsweise Speichermittel 141 zum Speichern von mindestens einer Matrix oder einer Zeile von gemessenen Sensordruckwerten. Weitere Speichermittel 141 zum Speichern weiterer Matrizen, die Filtermittel 142, die Druckberechnungsmittel 143 und die Darstellungsmittel 144 sind beispielsweise in der Auswertungseinheit 12 angeordnet, die beispielsweise einen programmierbaren Prozessor umfasst.

In der Figur 4 ist schematisch eine Ausführungsvariante der Messvorrichtung 1 dargestellt, in der die Messsonde 1', die im wesentlichen wie in der Ausführungsvariante gemäss Figur 3 ausgeführt ist, und die Auswertungseinheit 12 über eine kontaktlose Schnittstelle 13', beispielsweise eine Infrarot- oder eine Funkschnittstelle, miteinander verbunden sind. Die Auswertungseinheit 12 ist beispielsweise ein herkömmlicher Palmtop- oder Laptop-Computer, der zusätzlich mit einer kontaktlosen Schnittstelle 13' versehen ist. Die Filtermittel 142, die Druckberechnungsmittel 143 und die Darstellungsmittel 144 sind beispielsweise in der Auswertungseinheit 12 als programmierte Softwaremodule ausgeführt.

Die vorgeschlagene Messvorrichtung 1, respektive die vorgeschlagene Messsonde 1', ermöglicht die Messung des Augeninnendrucks durch eine einfache Applikation von mehreren Drucksensorelementen 111, ohne dass dazu eine Kraft oder eine Fläche exakt eingestellt werden muss, wobei kontinuierlich ein Druckprofil aufgezeichnet werden kann.

### Bezugszeichenliste

- 1: Vorrichtung zur Bestimmung des Augeninnendrucks (Messvorrichtung)
- 1': Messsonde
- 2: Auge
- 11: Drucksensor-Array
- 12: Auswertungseinheit
- 13: Kontaktbehaftete Schnittstelle
- 13': Kontaktlose Schnittstelle
- 14: Verarbeitungsmittel
- 15: Kommunikationsmodul
- 21: Hornhaut
- 22: Kontaktfläche (Applanationsfläche)
- 23: Tränenminiskus
- 111: Drucksensorelement
- 120: Anzeige
- 141: Speichermittel
- 142: Filtermittel
- 143: Druckberechnungsmittel
- 144: Darstellungsmittel
- p: Druckverteilungsprofil
- m: zweidimensionales Druckverteilungsprofil (Druckverteilungsmatrix)

## Patentansprüche

1. Vorrichtung (1) zur Bestimmung des Innendrucks eines Auges (2), insbesondere eines menschlichen Auges, welche mehrere Drucksensorelemente (111) umfasst, und welche Verarbeitungsmittel (14) zur Bestimmung des Augeninnendrucks aus den durch die einzelnen Drucksensorelemente (111) gemessenen Sensordruckwerten umfasst, welche Verarbeitungsmittel (14) so ausgebildet sind, dass sie den Augeninnendruck durch Division der Summe der gemessenen Sensordruckwerte durch die Anzahl der zur Summe beitragenden Drucksensorelemente (111) bestimmen, **dadurch gekennzeichnet,**
**dass** die Vorrichtung (1) Filtermittel (142) zum Ausschliessen von denjenigen gemessenen Sensordruckwerten von der Summierung umfasst, die mindestens mit einem definierten Differenzwert über einem Sensordruckwert (P) liegen, der von denjenigen Drucksensorelementen (111) gemessen wird, die im aufgesetzten Zustand der Vorrichtung (1) in einem inneren Bereich einer Kontaktfläche (22) liegen, auf welcher die Vorrichtung (1) mittelbar oder unmittelbar auf dem Auge (2) aufliegt.

2. Vorrichtung (1) gemäss Anspruch 1, **dadurch gekennzeichnet, dass** sie einen Drucksensor-Array (11) mit mindestens einer Zeile von den Drucksensorelementen (111) umfasst.

3. Vorrichtung (1) gemäss einem der Ansprüche 1 oder 2, **dadurch gekennzeichnet, dass** die Verarbeitungsmittel (14) so ausgebildet sind, dass sie den Augeninnendruck aus der Summe der gemessenen und gewichteten Sensordruckwerte und aus der Anzahl zur Summe beitragender Drucksensorelemente (111) bestimmen.

4. Vorrichtung (1) gemäss einem der Ansprüche 1 bis 3, **dadurch gekennzeichnet, dass** sie Speichermittel (141) zur Speicherung der gemessenen Sensordruckwerte und zur Speicherung mehrerer bestimmter Werte des Augeninnendrucks umfasst.

5. Vorrichtung (1) gemäss Anspruch 4, **dadurch gekennzeichnet, dass** sie Darstellungsmittel (144) zur grafischen Darstellung der gemessenen und/oder gespeicherten Sensordruckwerte und Augeninnendruckwerte umfasst.

6. Vorrichtung (1) gemäss einem der Ansprüche 1 bis 5, **dadurch gekennzeichnet, dass** sie Kommunikationsmittel (15) zur Femübermittlung der bestimmten Augeninnendruckwerte umfasst.

7. Vorrichtung (1) gemäss einem der Ansprüche 2 bis 6, **dadurch gekennzeichnet, dass** der Drucksensor-Array (11) als mikroelektromechanisches System ausgeführt ist.

8. Vorrichtung (1) gemäss einem der Ansprüche 1 bis 7, **dadurch gekennzeichnet, dass** sie als kompaktes, mobiles, tragbares Gerät ausgeführt ist.

9. Vorrichtung (1) gemäss einem der Ansprüche 2 bis 7, **dadurch gekennzeichnet, dass** der Drucksensor-Array (11) in eine Messsonde (1') eingebaut ist, die über eine kontaktbehaftete oder eine kontaktlose Schnittstelle (13, 13') mit einer Auswertungseinheit (12) verbunden ist.

10. Vorrichtungsanordnung, umfassend eine Messsonde (1') zur Erfassung von Messwerten für die Bestimmung des Innendrucks eines Auges (2), insbesondere eines menschlichen Auges, welche mehrere Drucksensorelemente (111) umfasst, und eine Auswertungseinheit (12), welche Verarbeitungsmittel (14) zur Bestimmung des Augeninnendrucks aus den durch die einzelnen Drucksensorelemente (111) gemessenen Sensordruckwerten umfasst, welche Verarbeitungsmittel (14) so ausgebildet sind, dass sie den Augeninnendruck durch Division der Summe der gemessenen Sensordruckwerte durch die Anzahl der zur Summe beitragenden Drucksensorelemente (111) bestimmen, **dadurch gekennzeichnet,**
**dass** die Messsonde (1') eine kontaktbehaftete oder eine kontaktlose Schnittstelle (13, 13') zur Übermittlung der durch die einzelnen Drucksensorelemente (111) gemessenen Sensordruckwerte an die Auswertungseinheit (12) umfasst, und
**dass** die Auswertungseinheit (12) Filtermittel (142) zum Ausschliessen von denjenigen gemessenen Sensordruckwerten von der Summierung umfasst, die mindestens mit einem definierten Differenzwert über einem Sensordruckwert (P) liegen, der von denjenigen Drucksensorelementen (111) gemessen wird, die im aufgesetzten Zustand der Messsonde (1') in einem inneren Bereich einer Kontaktfläche (22) liegen, auf welcher die Messsonde (1') mittelbar oder unmittelbar auf dem Auge (2) aufliegt.

11. Vorrichtungsanordnung gemäss Anspruch 10, **dadurch gekennzeichnet, dass** die Messsonde (1') einen Drucksensor-Array (11) mit mindestens einer Zeile von den Drucksensorelementen (111) umfasst.

## Claims

1. Device (1) for determining the internal pressure of an eye (2), in particular of a human eye, which device comprises a plurality of pressure sensor elements (111), and comprises processing means (14) for determining the intraocular pressure from the sensor pressure values measured by the individual pressure sensor elements (111), said processing means (14) being configured in such a way that they determine the intraocular pressure by dividing the sum of the measured sensor pressure values by the number of the pressure sensor elements (111) contributing to the sum, **characterized in that** the device (1) comprises filter means (142) for excluding, from the summation, those measured sensor pressure values which lie at least with a defined difference value above a sensor pressure value (P) which is measured by those pressure sensor elements (111) which, when the device (1) is in the applied state, lie in an inner region of a contact surface (22) on which the device (1) rests directly or indirectly on the eye (2).

2. Device (1) according to Claim 1, **characterized in that** it comprises a pressure sensor array (11) with at least one line of the pressure sensor elements (111).

3. Device (1) according to either of Claims 1 and 2, **characterized in that** the processing means (14) are configured in such a way that they determine the intraocular pressure from the sum of the measured and weighted sensor pressure values and from the number of pressure sensor elements (111) contributing to the sum.

4. Device (1) according to one of Claims 1 to 3, **characterized in that** it comprises memory means (141) for storing the measured sensor pressure values and for storing a plurality of determined values of the intraocular pressure.

5. Device (1) according to Claim 4, **characterized in that** it comprises presentation means (144) for graphic presentation of the measured and/or stored sensor pressure values and intraocular pressure values.

6. Device (1) according to one of Claims 1 to 5, **characterized in that** it comprises communication means (15) for remote transmission of the determined intraocular pressure values.

7. Device (1) according to one of Claims 2 to 6, **characterized in that** the pressure sensor array (11) is configured as a micro-electromechanical system.

8. Device (1) according to one of Claims 1 to 7, **characterized in that** it is configured as a compact, mobile, portable unit.

9. Device (1) according to one of Claims 2 to 7, **characterized in that** the pressure sensor array (11) is incorporated into a measuring probe (1') which is connected to an evaluation unit (12) via a contact interface or contactless interface (13, 13').

10. Device configuration, comprising a measuring probe (1') for recording measured values for determining the internal pressure of an eye (2), in particular of a human eye, which device configuration comprises a plurality of pressure sensor elements (111), and an evaluation unit (12) comprising processing means (14) for determining the intraocular pressure from the sensor pressure values measured by the individual pressure sensor elements (111), said processing means (14) being configured in such a way that they determine the intraocular pressure by dividing the sum of the measured sensor pressure values by the number of the pressure sensor elements (111) contributing to the sum,
**characterized in that** the measuring probe (1') comprises a contact interface or contactless interface (13, 13') for transmission of the sensor pressure values, measured by the individual pressure sensor elements (111), to the evaluation unit (12), and **in that** the evaluation unit (12) comprises filter means (142) for excluding, from the summation, those measured sensor pressure values which lie at least with a defined difference value above a sensor pressure value (P) which is measured by those pressure sensor elements (111) which, when the measuring probe (1') is in the applied state, lie in an inner region of a contact surface (22) on which the measuring probe (1') rests directly or indirectly on the eye (2).

11. Device configuration according to Claim 10, **characterized in that** the measuring probe (1') comprises a pressure sensor array (11) with at least one line of the pressure sensor elements (111).

## Revendications

1. Dispositif (1) pour déterminer la pression interne d'un oeil (2), en particulier d'un oeil humain, qui comprend plusieurs éléments capteurs de pression (111) et qui comprend des moyens de traitement (14) pour déterminer la pression interne de l'oeil à partir des valeurs de pression de capteur mesurées par les différents éléments capteurs de pression (111), lesquels moyens de traitement (14) sont conçus de manière telle qu'ils déterminent la pression interne de l'oeil par division de la somme des valeurs de pression de capteur mesurées par le nombre des éléments capteurs de pression (111) contribuant à la somme, **caractérisé en ce que** le dispositif (1) comprend des moyens de filtrage (142) pour exclure de l'addition les valeurs de pression de capteur mesurées qui dépassent d'une valeur différentielle définie une valeur de pression de capteur (P) qui est mesurée par les éléments capteurs de pression (111) qui, en position appliquée du dispositif (1), se trouvent dans une région intérieure d'une surface de contact (22) sur laquelle le dispositif (1) repose indirectement ou directement sur l'oeil (2).

2. Dispositif (1) selon la revendication 1, **caractérisé en ce qu'**il comprend un réseau (11) de capteurs de pression avec au moins une ligne des éléments capteurs de pression (111).

3. Dispositif (1) selon la revendication 1 ou 2, **caractérisé en ce que** les moyens de traitement (14) sont conçus de manière telle qu'ils déterminent la pression interne de l'oeil à partir de la somme des valeurs de pression de capteur mesurées et pondérées et à partir du nombre d'éléments capteurs de pression (111) contribuant à la somme.

4. Dispositif (1) selon une des revendications 1 à 3, **caractérisé en ce qu'**il comprend des moyens de mémoire (141) pour stocker les valeurs de pression de capteur mesurées et pour stocker plusieurs valeurs déterminées de la pression interne de l'oeil.

5. Dispositif (1) selon la revendication 4, **caractérisé en ce qu'**il comprend des moyens de représentation (144) pour représenter graphiquement les valeurs de pression de capteur et les valeurs de pression interne de l'oeil mesurées et / ou stockées.

6. Dispositif (1) selon une des revendications 1 à 5, **caractérisé en ce qu'**il comprend des moyens de communication (15) pour transmettre à distance les valeurs de pression interne de l'oeil déterminées.

7. Dispositif (1) selon une des revendications 2 à 6, **caractérisé en ce que** le réseau (11) de capteurs de pression est réalisé comme un microsystème électromécanique.

8. Dispositif (1) selon une des revendications 1 à 7, **caractérisé en ce qu'**il est réalisé comme un appareil portable, mobile, compact.

9. Dispositif (1) selon une des revendications 2 à 7, **caractérisé en ce que** le réseau (11) de capteurs de pression est intégré dans une sonde de mesure (1') qui est reliée à une unité d'évaluation (12) par l'intermédiaire d'une interface à contacts ou sans contact (13, 13').

10. Structure de dispositif comprenant une sonde de mesure (1') pour saisir des valeurs de mesure pour la détermination de la pression interne d'un oeil (2), en particulier d'un oeil humain, qui comprend plusieurs éléments capteurs de pression (111), et une unité d'évaluation (12), qui comprend des moyens de traitement (14) pour déterminer la pression interne de l'oeil à partir des valeurs de pression de capteur mesurées par les différents éléments capteurs de pression (111), lesquels moyens de traitement (14) sont conçus de manière telle qu'ils déterminent la pression interne de l'oeil par division de la somme des valeurs de pression de capteur mesurées par le nombre des éléments capteurs de pression (111) contribuant à la somme, **caractérisée en ce que** la sonde de mesure (1') comprend une inferface à contacts ou sans contact (13, 13') pour transmettre les valeurs de pression de capteur mesurées par les différents éléments capteurs de pression (111) à l'unité d'évaluation (12) et **en ce que** l'unité d'évaluation (12) comprend des moyens de filtrage (142) pour exclure de l'addition ces valeurs de pression de capteur mesurées qui dépassent d'une valeur différentielle définie une valeur de pression de capteur (P) qui est mesurée par les éléments capteurs de pression (111) qui, à l'état appliqué de la sonde de mesure (1'), se trouvent dans une région intérieure d'une surface de contact (22) sur laquelle la sonde de mesure (1') repose indirectement ou directement sur l'oeil (2).

11. Structure de dispositif selon la revendication 10, **caractérisée en ce que** la sonde de mesure (1') comprend un réseau (11) de capteurs de pression avec au moins une ligne des éléments capteurs de pression (111).
